# EUROPÄISCHE PATENTSCHRIFT

(11) **EP 3 177 340 B1**
(45) Veröffentlichungstag und Bekanntmachung des Hinweises auf die Patenterteilung: **29.05.2019**
(21) Anmeldenummer: 15753614.5
(22) Anmeldetag: 05.08.2015
(51) Int. Cl.: A61M 39/28, A61M 1/36, A61M 39/22

(54) **VERFAHREN ZUM AUSWASCHEN VON GASBLASEN IN EINEM EXTRAKORPORALEN BLUTKREISLAUF**
METHOD FOR WASHING OUT GAS BUBBLES IN AN EXTRACORPOREAL BLOOD CIRCUIT
PROCÉDÉ D'ÉLIMINATION PAR LAVAGE DE BULLES DE GAZ D'UN CIRCUIT DE SANG EXTRACORPOREL

(30) Priorität: 05.08.2014 DE 102014011673
(43) Veröffentlichungstag der Anmeldung: 14.06.2017
(73) Patentinhaber: Fresenius Medical Care Deutschland GmbH, 61352 Bad Homburg (DE)
(72) Erfinder: WOJKE, Ralf, 61348 Bad Homburg (DE); WIENEKE, Paul, 48149 Münster (DE)
(74) Vertreter: Herrmann, Uwe
(86) Internationale Anmeldenummer: PCT/EP2015/001617
(87) Internationale Veröffentlichungsnummer: WO 2016/020060

(56) Entgegenhaltungen:
- EP-A1- 1 837 046
- US-A- 5 951 870
- US-A1- 2011 213 289
- US-A1- 2014 110 319
- US-B1- 6 454 736

## Beschreibung

Die Erfindung betrifft ein Verfahren zum Auswaschen von Gasblasen aus einem Zielbereich eines extrakorporalen Blutkreislaufs, eine extrakorporale Blutbehandlungseinheit sowie ein Disposable für die extrakorporale Blutbehandlung.

Mikroblasen sind Gasblasen mit einem Durchmesser von wenigen µm, die aufgrund ihrer geringen Größe in der Regel nicht mehr sichtbar sind. Sie entstehen an verschiedenen Stellen und unter verschiedenen Bedingungen in extrakorporalen Blutkreisläufen, unter anderem durch Austreten von blutlöslichen Gasen oder Lufteintritt an kleinsten Undichtigkeiten im Unterdruckbereich des extrakorporalen Kreislaufs, und haften an der inneren Oberfläche des extrakorporalen Schlauchsystems oder der Blutbehandlungseinheit an. Ihre Auswirkungen, wenn sie losgelöst und in den menschlichen Körper infundiert werden, sind stärker als bisher vermutet. Beispielsweise wurden Mikroblasen in lebenswichtigen Organen wie Lunge, Herz und Hirn von Dialysepatienten nachgewiesen.

Beobachtungen an Dialysegeräten mit Hilfe eines Mikroblasendetektors lassen eine Reihe von möglichen Quellen für Mikroblasen erkennen. Unter anderem wurde beobachtet, dass in den ersten Minuten der Behandlung ein erhöhter Eintrag von Mikroblasen in den Patienten stattfindet. Am Anfang kann der mittlere Mikroblasenfluss um eine Größenordnung höher sein als während der restlichen Behandlung. Außerdem gibt es potentiell Luftansammlungen im extrakorporalen Kreislauf, die als Quelle und Reservoir für spätere Mikroblasenbildung dienen können. Luftansammlungen können beispielsweise im Dialysator, im Schlauchsegment der Blutpumpe, in der venösen Tropfkammer, in Heparin- und anderen Injektionsspritzen, in zuführenden Leitungen (z.B. in einem Heparinschlauch) und weiteren Schnittstellen (z.B. Luer-Verbindungen) vorhanden sein. Grundsätzlich begünstigt der Unterdruck im Saugbereich der Pumpe Lufteinträge. Ferner wurde auch während eines Druckhaltetests, bei dem die Dialysierflüssigkeit temporär nicht mehr erneuert wird und das Blut in den Dialysatorkapillaren nicht mehr mit frischer, entgaster Dialysierflüssigkeit umspült wird, eine erhöhte Anzahl an Mikroblasen beobachtet.

Mikroblasen können aufgrund ihrer geringen Größe und ihres geringen Auftriebes nur eingeschränkt in der venösen Kammer abgeschieden und von dem vorgeschriebenen Schutzsystem zur Vermeidung von Luftinfusion nur bedingt erkannt werden.

Der Erfindung liegt die Aufgabe zu Grunde, die Zahl an Mikroblasen in extrakorporalen Blutkreisläufen bzw. die Infusion von Mikroblasen in den Körper des Patienten zu verringern.

Vor diesem Hintergrund (siehe z. B. US 2011/213289 A1, US 2014/110319 A1, US 5 951 870 A, EP 1 837 046 A1, US 6 454 736 B1) schlägt die Erfindung ein Verfahren zum Auswaschen von Gasblasen aus einem Zielbereich eines extrakorporalen Blutkreislaufs vor, wobei der Zielbereich mit einer Spülflüssigkeit durchströmt wird, die durch einen Zulauf in den Zielbereich eintritt und diesen durch einen Ablauf wieder verlässt. Erfindungsgemäß ist vorgesehen, dass sich der Zulauf vom arteriellen Port und der Ablauf vom venösen Port des extrakorporalen Blutkreislaufs unterscheiden. Die Erfindung wird durch die Merkmale der unabhängigen Ansprüche 1, 9 definiert.

In einer Ausführungsform handelt es sich bei dem extrakorporalen Blutkreislauf um den einer Dialysemaschine. Der extrakorporale Blutkreislauf einer Dialysemaschine umfasst eine arterielle Leitung, eine Blutpumpe, einen Dialysator und eine venöse Leitung. In der arteriellen Leitung ist ein arterieller Port zum Anschluss an einen Patienten angeordnet. In der venösen Leitung ist ein venöser Port zum Anschluss an den Patienten angeordnet. Erfindungsgemäß verlässt die Spülflüssigkeit den Zielbereich nicht über den venösen Port, sondern über einen separaten Ablauf. Dadurch kann eine höhere Flexibilität mit Blick auf den Einsatzzeitpunkt des Verfahrens zum Auswaschen von Gasblasen erzielt werden. Da bekannte Verfahren aus dem Stand der Technik den venösen Port als Ablauf verwendeten bzw. einen Kurzschluss des venösen Ports verlangten, konnte ein Auswaschen von Gasblasen nicht erfolgen, während ein Patient an den Kreislauf angeschlossen war. Beispielsweise war ein Auswaschen von Gasblasen während einer Behandlungspause, beispielsweise während eines Druckhaltetests nicht möglich. Ferner ist ein Einsatz des Verfahrens auch in anderen extrakorporalen Behandlungssystemen denkbar, beispielsweise in Ultrafiltrationsgeräten und Herz-Lungen-Maschinen.

Der Zielbereich stellt einen Abschnitt des extrakorporalen Blutkreislaufs dar, der zwischen dem Zulauf und dem Ablauf liegt. Da sich der Zulauf vom arteriellen Port und der Ablauf vom venösen Port unterscheiden, umfasst der Zielbereich lediglich einen Teilabschnitt des extrakorporalen Blutkreislaufs. In unterschiedlichen Ausführungsformen können unterschiedliche Abschnitte bzw. Funktionsgruppen des extrakorporalen Blutkreislaufs außerhalb des Zielbereichs liegen. Beispielsweise kann eine vorzugsweise in der venösen Leitung angeordnete Blutpumpe außerhalb des Zielbereichs liegen.

Der Zielbereich wird vorzugsweise in Vorwärtsrichtung durchströmt. Die Vorwärtsrichtung ist so definiert, dass die Spülflüssigkeit in Richtung des venösen Ports strömt. Die Bezeichnungen 'stromaufwärts' und 'stromabwärts' beziehen sich im Rahmen der vorliegenden Erfindung auf die vorgesehene Strömungsrichtung im extrakorporalen Blutkreislauf. Vorzugsweise durchläuft die Spülflüssigkeit den Zielbereich während des gesamten Verfahrens immer in dieselbe Richtung. Vorzugsweise wird die Spülflüssigkeit während des Verfahrens nicht zirkuliert. Fallweise kann jedoch vorgesehen sein, dass zumindest zeitweise eine Zirkulation dahingehend stattfindet, dass verbrauchte Spülflüssigkeit, ggf. nach einer Aufbereitung, wiederverwendet wird.

In einer Ausführungsform ist der Ablauf in der venösen Leitung angeordnet. In einer Ausführungsform ist der Ablauf stromabwärts aller bestehenden Schnittstellen des extrakorporalen Blutkreislaufs angeordnet. Beispiele bestehender Schnittstellen umfassen einen Prädilutions-Port, einen Postdilutions-Port, einen Port zur Zugabe von Anitkoagulantien (z.B. Heparin oder Citrat), einen Port für die Blutentnahme, einen Port für arterielle oder venöse Medikamentenzugabe oder dergleichen. Der arterielle und venöse Port werden nicht als Schnittstelle im Sinne dieser Ausführungsform verstanden. Sofern der Ablauf stromabwärts aller bestehenden Schnittstellen des extrakorporalen Blutkreislaufs angeordnet ist, kann ein möglichst großer Teil des extrakorporalen Blutkreislaufs von Mikroblasen befreit werden, und im Falle eines gleichzeitig zugegebenen Antikoagulans benetzt werden. Vorzugsweise befindet sich der Ablauf nahe dem venösen Port. Es ist allerdings vorteilhaft, den Ablauf stromaufwärts etwaiger Schutzsysteme gegen Luftinjektion, wie z.B. eines Luftblasendetektors anzuordnen.

In einer Ausführungsform ist der Zulauf in der arteriellen Leitung angeordnet. In einer Ausführungsform ist der Zulauf stromaufwärts der Blutpumpe angeordnet. Alternativ kann der Zulauf stromabwärts der Blutpumpe angeordnet sein. Generell kann der Zugang des Substituatstroms nahe an dem arteriellen Port liegen, um einen möglichst großen Teil des Schlauchsystems von Mikroblasen befreien zu können. Im Falle einer gewünschten intermittierenden Spülung mit einem Antikoagulans sollte der Zugang des Substituatstroms vorzugsweise stromauf zum Schlauch zur Zugabe des Antikoagulans liegen, um das gleichzeitig zugegebene Antikoagulans mitzureißen und einen möglichst großen Teil des Schauchsystems zu benetzen. Daher liegt der Zugang in einer Ausführungsform stromaufwärts der Blutpumpe. Alternativ ist auch eine Anordnung des Zulaufs stromabwärts der Blutpumpe, und gegebenenfalls stromaufwärts einer Schnittstelle für die Antikoagulanszugabe (z.B. Heparin) denkbar.

In einer Ausführungsform werden bestehende Schnittstellen als Zulauf genützt. Beispiele von bestehenden Schnittstellen, die als Zulauf genützt werden können, umfassen einen Prädilutions-Port oder einen Port zur Zugabe von Anitkoagulantien. In einer Ausführungsform weist der extrakorporale Blutkreislauf einen als separate Schnittstelle ausgebildeten Zulauf auf. In einer denkbaren Ausführungsform kann beispielsweise vorgesehen sein, dass ein zusätzlicher Zulauf stromaufwärts der Blutpumpe angeordnet wird, sodass auch der Bereich um die Blutpumpe herum gespült bzw. von Mikroblasen befreit werden kann.

Der Zielbereich umfasst vorzugsweise solche Abschnitte des extrakorporalen Blutkreislaufs, in denen eine Gasblasenbildung oder -ablagerung häufig auftritt. Sofern der Zulauf in der arteriellen Leitung und der Ablauf in der venösen Leitung angeordnet ist, umfasst der Zielbereich im Falle einer Dialysemaschine jedenfalls den Dialysator. Wenn der Zulauf stromaufwärts der Blutpumpe angeordnet ist, umfasst der Zielbereich ferner die Blutpumpe. Weitere Abschnitte, die vom Zielbereich erfasst sein können, umfassen die venöse Tropfkammer, Heparin- und anderen Injektionsspritzen, oder im allgemeinen zuführende Leitungen (z.B. konnektiert über eine Luer-Verbindung) wie beispielsweise einen Heparinschlauch.

In einer Ausführungsform umfasst der extrakorporale Blutkreislauf eine arterielle und/oder eine venöse Klemme. Die arterielle Klemme ist vorzugsweise zwischen arteriellem Port und Zulauf angeordnet. Die venöse Klemme ist vorzugsweise zwischen Ablauf und venösem Port angeordnet. Auf eine arterielle Klemme kann beispielsweise dann verzichtet werden, sofern der Zulauf stromabwärts der Blutpumpe angeordnet ist. Denn auch eine stehende Blutpumpe kann eine entsprechende Sperrwirkung entfalten. Der Begriff einer Klemme umfasst vorliegend auch ein Sperrventil.

In einer Ausführungsform ist der Zulauf und/oder der Ablauf anhand einer Klemme verschließbar. In einer Ausführungsform ist der Zulauf und/oder der Ablauf anhand eines Dreiwegeventils mit dem extrakorporalen Blutkreislauf verbunden. Unter einer Klemme ist ein Mittel zu verstehen, das geeignet ist, die arterielle bzw. venöse Leitung zu blockieren. Anhand eines Dreiwegeventils kann alternativ am stromaufwärtigen Ende des Zielbereichs der arterielle Port oder der Zulauf vom Zielbereich und am stromabwärtigen Ende des Zielbereichs der venöse Port oder der Ablauf vom Zielbereich abgekoppelt werden.

In einer Ausführungsform ist die Flussrate und/oder der Druck der Spülflüssigkeit im extrakorporalen Blutkreislauf während des Spülvorganges wenigstens zeitweise inkonstant. So kann eine effizientere Ablösung von Mikroblasen von den Wänden des extrakorporalen Blutkreislaufs erreicht werden. Die Variation der Flussrate und/oder des Drucks kann durch eine Änderung der Förderrate einer Pumpe oder durch Ansteuerung einer Klemme oder eines Ventils erfolgen. In einer Ausführungsform wird die Flussrate und/oder der Druck der Spülflüssigkeit in Stößen erhöht und reduziert. Die Stöße können einen kontinuierlichen oder abrupten Aufbau und einen kontinuierlichen oder abrupten Abfall der Flussrate und/oder des Drucks der Spülflüssigkeit zeigen. Die Amplitude der Stöße kann so gewählt werden, dass die Flussrate und/oder der Drucks der Spülflüssigkeit zwischen Tälern und Bergen um wenigstens den Faktor 1,3, den Faktor 1,5 oder den Faktor 2 variiert.

In einer Ausführungsform liegt die Flussrate der Spülflüssigkeit im extrakorporalen Blutkreislauf während des Spülvorganges wenigstens zeitweise außerhalb eines Bereichs möglicher Flussraten. Letzteres Merkmal kann bedeuten, dass die Flussrate der Spülflüssigkeit im extrakorporalen Blutkreislauf während des Spülvorganges wenigstens zeitweise oberhalb eines Bereichs möglicher Flussraten liegt, der während der Behandlung für das Blut angewendet wird. Ferner besteht beispielsweise die Möglichkeit der Flussumkehr. In einer Ausführungsform ist die Flussrate der Spülflüssigkeit im extrakorporalen Blutkreislauf während des Spülvorganges wenigstens zeitweise größer als 550 ml/min oder größer 700 ml/min. In vielen Fällen wird während einer extrakorporalen Blutbehandlung ein Fluss von zwischen 200 ml/min und 550 ml/min gewählt, insbesondere dann, wenn Schlauchsysteme mit einem Innendurchmesser von zwischen 3 und 5 mm verwendet werden. Eine höhere Flussrate während des Spülvorganges kann zu einer besseren Ablösung von Gasblasen führen.

In einer Ausführungsform kann das Verfahren im Rahmen des Primings des extrakorporalen Blutkreislaufs durchgeführt werden, und beispielsweise den abschließenden Schritt des Primingvorgangs darstellen.

In einer Ausführungsform wird das Verfahren während einer Behandlungsunterbrechung, insbesondere während eines Druckhaltetests durchgeführt. Das Verfahren kann manuell oder automatisch ausgelöst werden. Beispiele für Auslösebedingungen umfassen eine periodische Auslösung (z.B. bei Intervallgrößen von 30 Minuten bis 2 Stunden), oder eine Auslösung in Reaktion auf bestimmte Sensormeldungen (z.B. Messwert des Blasendetektors überschreitet einen Grenzwert) oder Ereignisse (z.B. Beginn eines Druckhaltetests). Die Spülung kann über einen vorbestimmten Zeitraum erfolgen (z.B. mit einer Dauer von zwischen 1 und 5 Minuten), oder auf der Grundlage unterschiedlicher Parameter manuell oder automatisch bestimmt werden. Auch die Kopplung an die Dauer eines anderen Prozesses wie beispielsweise eines Druckhaltetests ist denkbar.

Das Verfahren umfasst die folgenden Schritte: (a) Schließen einer arteriellen Klemme und Öffnen des Zulaufs; (b) Fördern eines ersten Volumens an Spülflüssigkeit in den Zielbereich, wobei das erste Volumen dem Volumen des Zielbereichs im Wesentlichen entspricht; (c) Schließen einer venösen Klemme und Öffnen des Ablaufs; (d) Durchspülen des Zielbereichs mit der Spülflüssigkeit; (e) Öffnen der arteriellen Klemme und Schließen des Zulaufs; (f) Ableiten eines zweiten Volumens an Spülflüssigkeit aus dem Zielbereich, wobei das zweite Volumen dem Volumen des Zielbereichs im Wesentlichen entspricht; und (g) Schließen des Ablaufs und Öffnen der venösen Klemme. Der Zulauf kann stromaufwärts der Blutpumpe angeordnet sein. Die Förderung der Spülflüssigkeit in Schritten (b), (d) und (f) erfolgt vorzugsweise anhand der Blutpumpe.

In einer Ausführungsform umfasst das Verfahren die folgenden Schritte: (a') Anhalten der Blutpumpe und Öffnen des Zulaufs; (b') Fördern eines ersten Volumens an Spülflüssigkeit in den Zielbereich, wobei das erste Volumen dem Volumen des Zielbereichs im Wesentlichen entspricht; (c') Schließen einer venösen Klemme und Öffnen des Ablaufs; (d') Durchspülen des Zielbereichs mit der Spülflüssigkeit; (e') Schließen des Zulaufs und Starten der Blutpumpe; (f') Ableiten eines zweiten Volumens an Spülflüssigkeit aus dem Zielbereich, wobei das zweite Volumen dem Volumen des Zielbereichs im Wesentlichen entspricht; und (g') Schließen des Ablaufs und Öffnen der venösen Klemme. In dieser Ausführungsform ist der Zulauf vorzugsweise stromabwärts der Blutpumpe angeordnet. Die Förderung der Spülflüssigkeit in Schritten (b') und (d') erfolgt vorzugsweise anhand einer separaten Spülpumpe. Die Förderung der Spülflüssigkeit in Schritt (f') erfolgt vorzugsweise anhand der Blutpumpe.

Die Verfahrensschritte erfolgen vorzugsweise in der genannten Reihenfolge (a) bis (g) bzw. (a') bis (g'). In einer Ausführungsform kann das Merkmal, dass das erste bzw. zweite Volumen dem Volumen des Zielbereichs ,im Wesentlichen' entspricht, eine maximale Abweichung von ±20% oder ±10% bezeichnen. Dies spiegelt wieder, dass oftmals keine genaue Entsprechung erforderlich ist, eine ungefähre Entsprechung aber vorteilhaft ist. Sofern eine Abweichung stattfindet, kann die Menge der zugeführter Spülflüssigkeit oder abgeleiteten Blutes bei der Ermittlung von einer Steuereinheit erfasst und verarbeitet werden. Dies kann in weiterer Folge beispielsweise zu einer Berücksichtigung bei der Ermittlung behandlungsspezifischer Parameter, insbesondere bei der Ermittlung des Ultrafiltrationsvolumens führen. Das Schließen und Öffnen in Schritten (a) und (e) bzw. (a') und (e') sowie in Schritten (c) und (g) bzw. (c') und (g') kann durch ein Umschalten eines Dreiwegeventils erfolgen.

Die Erfindung betrifft vor dem eingangs geschilderten Hintergund ferner eine extrakorporale Blutbehandlungseinheit mit einem extrakorporalen Blutkreislauf und einer Steuereinheit, wobei erfindungsgemäß vorgesehen ist, dass der extrakorporale Blutkreislauf einen Zulauf und einen Ablauf für Spülflüssigkeit aufweist, wobei sich der Zulauf vom arteriellen Port und der Ablauf vom venösen Port des extrakorporalen Blutkreislaufs unterscheiden, und wobei die Steuereinheit ausgebildet ist, um ein erfindungsgemäßes Verfahren durchzuführen.

Das Merkmal, dass die Steuereinheit ausgebildet ist, um ein Verfahren gemäß einem der vorhergehenden Ansprüche durchzuführen, bedeutet, dass in der Steuereinheit eine entsprechende Routine hinterlegt ist und dass die Steuereinheit mit den notwendigen Aktoren (z.B. Pumpen und Ventile) verbunden ist, die für die Durchführung des Verfahrens erforderlich sind. Die extrakorporale Blutbehandlungseinheit kann bauliche Merkmale umfassen, die im Zusammenhang mit dem erfindungsgemäßen Verfahrens beschrieben wurden.

In einer Ausführungsform handelt es sich bei der extrakoproralen Blutbehandlungseinheit um eine Dialysemaschine. Die Dialysemaschine kann zur Durchführung einer Hämodialyse, einer Hämodiafiltration, einer Hämofiltration und/oder einer Ultrafiltration (reiner Flüssigkeitsentzug) geeignet und bestimmt sein. Ferner kann es sich bei der erfindungsgemäßen extrakorporalen Blutbehandlungseinheit um ein anderes Behandlungssystemen handeln, beispielsweise ein Ultrafiltrationsgerät oder eine Herz-Lungen-Maschine.

In einer Ausführungsform umfasst die Blutbehandlungseinheit ein Leitungssystem für Spülflüssigkeit, das so an den extrakorporalen Blutkreislauf angeschlossen ist, dass Spülflüssigkeit durch einen Zulauf in den Zielbereich eintreten und diesen durch einen Ablauf wieder verlassen kann, wobei das Leitungssystem vorzugsweise eine Spülpumpe umfasst, die stromaufwärts des Zulaufs im Leitungssystem angeordnet ist.

In einer Ausführungsform umfasst das Leitungssystem ein Reservoir für Spülflüssigkeit oder eine Zubereitungseinheit für Spülflüssigkeit, das oder die stromaufwärts des Zulaufs im Leitungssystem angeordnet ist. Die Steuereinheit kann mit der Spülpumpe in Verbindung stehen und so ausgebildet sein, dass diese die Spülpumpe im Rahmen des erfindungsgemäßen Verfahrens ansteuert, um eine Spülung des Zielbereichs zu bewirken.

In einer Ausführungsform ist die Steuereinheit so ausgebildet, dass sie das Verfahren automatisch im Rahmen des Primings des extrakorporalen Blutkreislaufs auslöst. Beispielsweise kann das Verfahren den abschließenden Primingschritt darstellen.

In einer Ausführungsform ist die Steuereinheit so ausgebildet, dass sie die Behandlung vor Durchführung des Verfahrens unterbricht und/oder dass sie das Verfahren während einer Unterbrechung der Behandlung auslöst und/oder dass sie die Behandlung nach Durchführung des Verfahrens automatisch fortsetzt. Beispielsweise kann die Steuereinheit ausgebildet sein, die Behandlung eigens zur Durchführung des Verfahrens zu unterbrechen. Die Unterbrechung erfolgt beispielsweise periodisch (z.B. bei Intervallgrößen von 30 Minuten bis 2 Stunden) oder in Reaktion auf bestimmte Sensormeldungen, beispielsweise Meldungen eines in der venösen Leitung angeordneten Gasblasensensors. Ferner kann die Steuereinheit so ausgebildet sein, dass das Verfahren dann ausgelöst wird, wenn die Behandlung ohnehin unterbrochen ist. Beispielsweise kann das Verfahren während eines Druckhaltetests ausgelöst werden. Die Dauer des Verfahrens kann beispielsweise der Dauer eines gleichzeitig durchgeführten Druckhaltetests entsprechen (z.B. zwischen 1 und 5 Minuten).

In einer Ausführungsform ist die Steuereinheit so ausgebildet, dass sie die Menge von durch das Verfahren zugeführter Spülflüssigkeit und/oder durch das Verfahren abgeleiteten Blutes bei der Ermittlung behandlungsspezifischer Parameter, insbesondere bei der Ermittlung des Ultrafiltrationsvolumens berücksichtigt.

In einer Ausführungsform ist die Steuereinheit so ausgebildet, dass nach Abschluss des Verfahrens die Fördergeschwindigkeit für Blut langsam erhöht wird. So kann erreicht werden, dass zu Beginn der Blutbehandlung weniger Mikroblasen in das Blut ausgelöst werden. Beispielsweise kann die Fördergeschwindigkeit auf einer ggf. linearen Rampe mit einer Steigerung von nicht mehr als 300 ml/min² oder 200 ml/min² oder 150 ml/min² erhöht werden.

Bei der Blutpumpe und/oder der Spülpumpe kann es sich um eine peristaltische Pumpe handeln. Bei der Spülflüssigkeit kann es sich beispielsweise um eine Substitutions- bzw. Priminglösung handeln. Beispiele umfassen physiologische Kochsalzlösung und Ringer Lösung. Gegebenenfalls kann die Lösung ferner antikoagulative Mittel wie beispielsweise Heparin enthalten.

Des Weiteren betrifft diese Offenbarung vor dem eingangs geschilderten Hintergund ein Disposable für die extrakorporale Blutbehandlung, wobei das Disposable eine arterielle Leitung, Elemente einer Blutpumpe, eine Blutbehandlungseinheit und eine venöse Leitung umfasst. Es ist vorgesehen, dass das Disposable in der arteriellen Leitung eine Schnittstelle für den Zulauf von Spülflüssigkeit und in der venösen Leitung eine Schnittstelle für den Ablauf von Spülflüssigkeit aufweist, wobei sich die Schnittstelle für den Zulauf vom arteriellen Port und die Schnittstelle für den Ablauf vom venösen Port des Schlauchsets unterscheiden.

In einer Ausführungsform handelt es sich bei dem Disposable um eines für die Dialysebehandlung und bei dem Blutbehandlungsaggregat um einen Dialysator.

Ferner kann es sich bei dem Disposable beispielsweise um ein Fluidsystem eines Ultrafiltrationsgeräts oder einer Herz-Lungen-Maschine handeln.

Die Blutpumpe kann vollständig im Disposable integriert sein, allerdings bietet es sich aus Kostengünden an, die Pumpe maschinenseitig anzuordnen und nur gewisse Elemente der Pumpe am Disposable anzuordnen, die mit weiteren und maschinenseitig angeordneten Elementen der Pumpe wechselwirken und gemeinsam eine Pumpe bilden. Ein Beispiel ist eine peristaltische Pumpe, wobei am Disposable ein elastisch verformbares Schlauchsegment angeordnet sein kann, in welches maschinenseitig angeordnete Aktoren einer Pumpe eingreifen sollen.

Vorzugsweise umfasst das Disposable ein Blutschlauchset, wobei die arterielle und die venöse Leitung zumindest abschnittsweise als Blutschlauch ausgebildet sind. Beispielsweise beträgt der Innendurchmesser der Blutschläuche zwischen 3 und 5 mm. Das Disposable kann zumindest teilweise aus Kunststoff gefertigt sein. Beim Blutbehandlungsaggregat handelt es sich vorzugsweise um einen Hohlfaserdialysator.

In einer Ausführungform weist die Schnittstelle für den Zulauf und/oder die Schnittstelle für den Ablauf von Spülflüssigkeit Elemente eines mechanischen Verbindungssystems auf. Diese Verbindungselementen umfassen vorzugsweise einen weiblichen Kegel eines Luer-Locks oder Luer-Ansatzes. Dadurch wird das Disposable vielseitig einsetzbar, da ein männlicher Luer-Kegel an vielen maschinenseitigen Anschlüssen von bestehenden Geräten zu finden ist.

Das Disposable kann in einem erfindungsgemäßen Verfahren bzw. einer erfindungsgemäßen Blutbehandlungseinheit zu Einsatz kommen.

In einer Ausführungsform ist die Schnittstelle für den Ablauf von Spülflüssigkeit stromabwärts aller anderen Schnittstellen des Disposables angeordnet. Beispiele bestehender Schnittstellen umfassen eine Schnittstelle für eine Prädilution, eine Schnittstelle für eine Postdilution, eine Schnittstelle zur Zugabe von Antikoagulantien, eine Schnittstelle für die Blutentnahme, Schnittstellen für den arterielle oder venöse Medikamentengabe oder dergleichen. Der arterielle und venöse Port werden nicht als Schnittstelle im Sinne dieser Ausführungsform verstanden.

In einer Ausführungsform ist die Schnittstelle für den Zulauf von Spülflüssigkeit stromaufwärts des Pumpsegments angeordnet. Alternativ kann vorgesehen sein, dass die Schnittstelle für den Zulauf von Spülflüssigkeit stromabwärts des Pumpsegments angeordnet ist. In einer Ausführungsform werden bestehende Schnittstellen als Schnittstelle für den Zulauf von Spülflüssigkeit genützt. Beispiele umfassen eine Schnittstelle für die Prädilution oder eine Schnittstelle zur Zugabe von Antikoagulantien. In einer anderen Ausführungsform weist das Disposable eine separate Schnittstelle für den Zulauf von Spülflüssigkeit auf.

In einer Ausführungsform umfasst der extrakorporale Blutkreislauf eine arterielle und/oder venöse Klemme, wobei die Elemente der arteriellen Klemme vorzugsweise zwischen arteriellem Port und Schnittstelle für den Zulauf von Spülflüssigkeit und die venöse Klemme vorzugsweise zwischen Schnittstelle für den Ablauf von Spülflüssigkeit und venösem Port angeordnet sind.

In einer Ausführungsform ist die Schnittstelle für den Zulauf und/oder Ablauf von Spülflüssigkeit anhand einer Klemme verschließbar oder anhand eines Dreiwegeventils mit der arteriellen bzw. venösen Leitung verbunden. Die Klemme kann vollständig im Disposable integriert sein. Ein Beispiel umfasst ein Sperrventil. Allerdings können auch nur gewisse Elemente der Klemme am Disposable angeordnet sein, die mit weiteren und maschinenseitig angeordneten Elementen der Klemme wechselwirken und gemeinsam eine Klemme bilden. Ein Beispiel ist ein elastisch verformbares (Schlauch-)segment am Disposable im Zusammenwirken mit einem maschinenseitig angeordneten Aktor.

Weitere Einzelheiten und Vorteile ergeben sich aus den nachfolgend beschriebenen Figuren und Ausführungsbeispielen. In den Figuren zeigen:
- Figur 1:: eine schematische Darstellung von Fluidkreisläufen einer Dialysemaschine aus dem Stand der Technik;
- Figur 2:: eine schematische Darstellung von Fluidkreisläufen gemäß einer Dialysemaschine gemäß einer ersten Ausführungsform der Erfindung; und
- Figur 3:: eine schematische Darstellung von Fluidkreisläufen gemäß einer Dialysemaschine gemäß einer zweiten Ausführungsform der Erfindung.

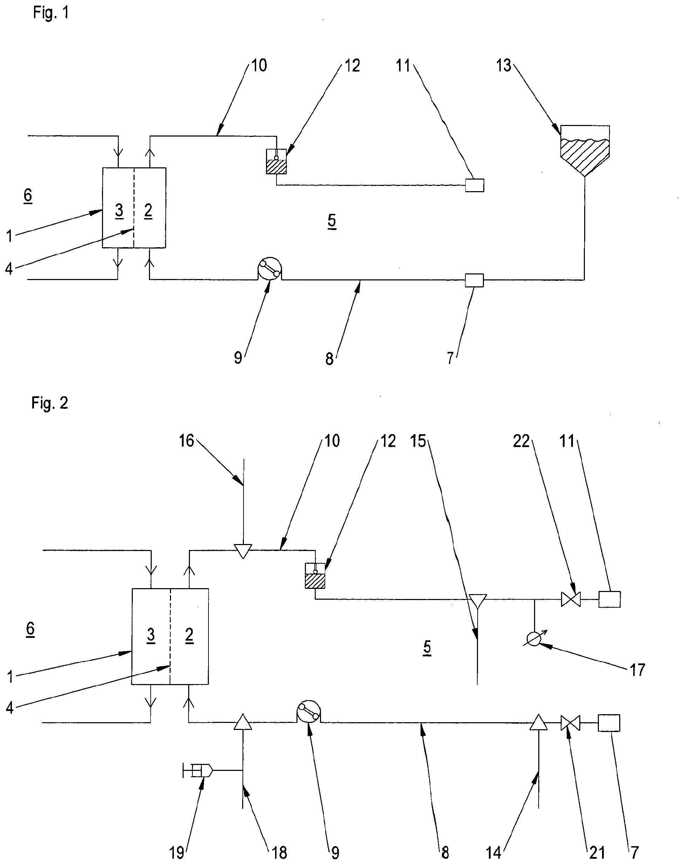

Figur 1 zeigt Fluidkreisläufe einer Dialysemaschine aus dem Stand der Technik. Die Dialysemaschine weist einen Dialysator 1 auf, der eine Blutkammer 2 und eine Dialysierflüssigkeitskammer 3 aufweist, die durch eine Membran 4 voneinander getrennt sind. Die Blutkammer 2 ist Bestandteil eines extrakorporalen Blutkreislaufs 5. Die Dialysierflüssigkeitskammer 3 ist Bestandteil eines Dialysierflüssigkeitskreislaufs 6.

Der Blutkreislauf 5 weist einen arteriellen Port 7 auf, der über eine arterielle Leitung 8 mit der Blutkammer 2 verbunden ist. In der arteriellen Leitung 8 sitzt eine Blutpumpe 9. Das venöse Ende der Blutkammer 2 ist anhand der venösen Leitung 10 mit dem venösen Port 11 verbunden. In der venösen Leitung befindet sich eine Tropfkammer 12.

Während des Füllens und Spülens (Priming) des Blutkreislaufs sind der arterielle und venöse Port nicht mit dem Patienten verbunden. Der arterielle Port ist mit einem Spülflüssigkeitsreservoir 13 verbunden und der venöse Port stellt einen Ablauf für Spülflüssigkeit dar. Nach dem Befüllen des Blutkreislaufs mit Spülflüssigkeit erfolgt eine Spülphase, wobei Spülflüssigkeit aus dem venösen Port austritt.

Ein Spülen des Blutkreislaufs kann so nicht erfolgen, während ein Patient an den Kreislauf angeschlossen ist, da der arterielle Port und der venöse Port nicht für die Spülung zur Verfügung stehen.

Figur 2 zeigt Fluidkreisläufe einer Dialysemaschine gemäß einer ersten Ausführungsform der Erfindung. Bereits aus Figur 1 bekannte Bauteile sind mit entsprechenden Bezugszeichen versehen.

Im Unterschied zum Stand der Technik weist der extrakorporale Blutkreislauf 5 hier ferner einen separaten Zulauf 14 und einen separaten Ablauf 15 für Spülflüssigkeit auf. So wird ermöglicht, dass die Spülflüssigkeit einen zwischen Zulauf und Ablauf liegenden Zielbereich für eine Spülung nicht über den venösen Port 11, sondern über den separaten Ablauf 15 verlassen kann. Ferner kann Spülflüssigkeit über einen separaten Zulauf in den Kreislauf eintreten.

Der Ablauf 15 ist in der venösen Leitung 10 stromabwärts aller bestehenden Schnittstellen des extrakorporalen Blutkreislaufs angeordnet. Beispielhaft ist in der Figur als Schnittstelle ein Postdilutions-Port 16 dargestellt. Zwischen Ablauf und venösem Port befindet sich ein Luftblasendetektor 17. Der Zulauf ist in der arteriellen Leitung stromaufwärts der Blutpumpe angeordnet und stellt einen separaten Zugang dar. Als weitere Schnittstelle umfasst der Blutkreislauf einen Prädilutions-Port 18 mit integrierter Heparinzuleitung 19. Der extrakorporale Blutkreislauf umfasst ferner eine arterielle Klemme 20 und eine venöse Klemme 21. Die arterielle Klemme ist zwischen arteriellem Port 7 und Zulauf 14 angeordnet. Die venöse Klemme ist zwischen Ablauf 15 und venösem Port 11 angeordnet. Sowohl der Zulauf 14 als auch der Ablauf 15 sind anhand eines Dreiwegeventils mit dem extrakorporalen Blutkreislauf 15 verbunden.

Durch die Anordnung des zusätzlichen Ablaufs und Zulaufs sowie durch die weiteren Bauteile des erfindungsgemäßen Dialysegerätes kann eine höhere Flexibilität mit Blick auf den Einsatzzeitpunkt des Verfahrens zum Auswaschen von Gasblasen erzielt werden. Beispielsweise ist ein Auswaschen von Gasblasen während einer Behandlungspause, beispielsweise während eines Druckhaltetests möglich. Beispielsweise kann eine Spülung vor oder während der Behandlung die folgenden Schritte aufweisen:
(a) Schließen der arteriellen Klemme und Öffnen des Zulaufs;
(b) Fördern eines ersten Volumens an Spülflüssigkeit in den zwischen Zulauf und Ablauf liegenden Zielbereich, wobei das erste Volumen dem Volumen des Zielbereichs im Wesentlichen entspricht;
(c) Schließen der venösen Klemme und Öffnen des Ablaufs;
(d) Durchspülen des Zielbereichs mit der Spülflüssigkeit;
(e) Öffnen der arteriellen Klemme und Schließen des Zulaufs;
(f) Ableiten eines zweiten Volumens an Spülflüssigkeit aus dem Zielbereich, wobei das zweite Volumen dem Volumen des Zielbereichs im Wesentlichen entspricht; und
(g) Schließen des Ablaufs und Öffnen der venösen Klemme.

Die Förderung der Spülflüssigkeit in Schritten (b), (d) und (f) erfolgt anhand der Blutpumpe. Die Förderung der Spülflüssigkeit in das System erfolgt anhand einer in den Figuren nicht dargestellten separaten Spülpumpe, die im maschinenseitigen Versorgungssystem für die Spülflüssigkeit angeordnet ist.

Sofern wie im gezeigten Ausführungsbeispiel der Zugang des Substituatstroms vor der Blutpumpe liegt, kann das Ausspülen der Mikroblasen durchgeführt werden, in dem Substituatpumpe und Blutpumpe mit gleicher Förderrate laufen. Da das Substituat dann vollständig von der Blutpumpe mit gleicher Förderrate weiter befördert wird, kann kein Blut mehr in das Schlauchsystem gezogen werden, sodass auf eine arterielle Klemme auch verzichtet werden kann. Alternativ ermöglicht eine arterielle Klemme das vorübergehende Abklemmen des Blutstroms und große Freiheiten bezüglich Variation der Förderraten von Blutpumpe und Substituatpumpe zum Erzeugen von Druckpulsen und Volumenstrompulsen, die geeignet sein können, um makroskopische Blasen und Mikroblasen abzulösen und mitzureißen.

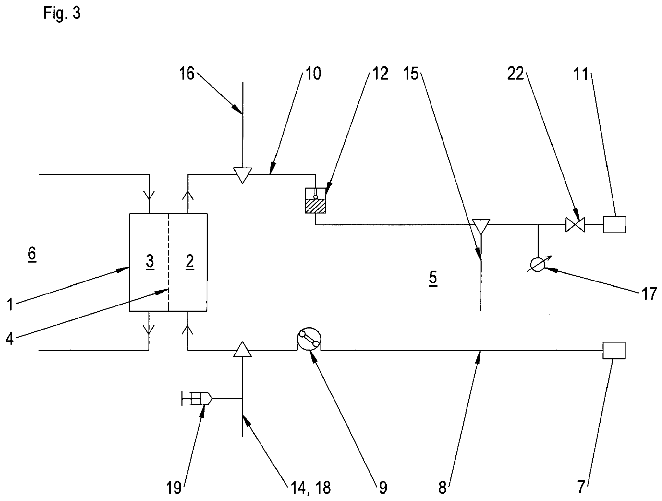

Figur 3 zeigt Fluidkreisläufe einer Dialysemaschine gemäß einer weiteren Ausführungsform der Erfindung. Bereits aus Figuren 1 und 2 bekannte Bauteile sind mit entsprechenden Bezugszeichen versehen.

Diese Ausführungsform unterscheidet sich von der in Figur 1 gezeigten Ausführungsform dadurch, dass der Zulauf 14 in der arteriellen Leitung stromabwärts der Blutpumpe angeordnet ist und dem Prädilutions-Port 18 mit integrierter Heparinzuleitung 19 entspricht. Auf eine arterielle Klemme wurde verzichtet. Beispielsweise kann eine Spülung vor oder während der Behandlung unter Verwendung dieser Ausführungsform der Erfindung die folgenden Schritte aufweisen:
(a') Anhalten der Blutpumpe und Öffnen des Zulaufs;
(b') Fördern eines ersten Volumens an Spülflüssigkeit in den zwischen Zulauf und Ablauf liegenden Zielbereich, wobei das erste Volumen dem Volumen des Zielbereichs im Wesentlichen entspricht;
(c') Schließen der venösen Klemme und Öffnen des Ablaufs;
(d') Durchspülen des Zielbereichs mit der Spülflüssigkeit;
(e') Schließen des Zulaufs und Starten der Blutpumpe;
(f') Ableiten eines zweiten Volumens an Spülflüssigkeit aus dem Zielbereich, wobei das zweite Volumen dem Volumen des Zielbereichs im Wesentlichen entspricht; und
(g') Schließen des Ablaufs und Öffnen der venösen Klemme.

Die Förderung der Spülflüssigkeit in Schritten (b') und (d') erfolgt anhand einer in den Figuren nicht dargestellten separaten Spülpumpe, die im maschinenseitigen Versorgungssystem für die Spülflüssigkeit angeordnet ist. Die Förderung der Spülflüssigkeit in Schritt (f') erfolgt anhand der Blutpumpe 9.

Sofern anhand einer Dialysemaschine gemäß der Erfindung ein Auswaschen des Zielbereichs während der Durchführung eines Druckhaltetests im Dialysierflüssigkeitskreislauf erfolgt, bestehen in beiden gezeigten Ausführungsformen zwei Möglichkeiten der Verfahrensführung, nämlich eine zeitliche und eine räumliche Entkoppelung.

Zeitliches Entkoppeln bedeutet dass bereits vor dem unmittelbaren Beginn des Druckhaltetests die Spülung eingeleitet wird und Substitutionsflüssigkeit als Spülflüssigkeit in den Zielbereich gespült wird. Räumliches Entkoppeln bedeutet, dass ein weiteres Gefäß, beispielsweise ein Beutel zur Zwischenspeicherung von Substituat verwendet wird.

Während der zeitlichen Entkoppelung wird der Blutstrom im extrakorporalen Blutkreislauf gestoppt und durch Zugabe der Spüllösung in die arterielle Leitung ersetzt. Die Spüllösung wird bei noch laufender Blutpumpe solange eingefüllt, bis die Grenzschicht Substituat/Blut körpernah etwa auf Höhe der venösen Kammer detektierbar ist, beispielsweise durch einen optischen Detektor oder Farbsensor. Dann wird der Dialysierflüssigkeitskreislauf für den Druckhaltetest gestoppt. Dann wird die venöse Klemme geschlossen und der Ablauf geöffnet. Während des Druckhaltetests wird der Zielbereich anhand eines erfindungsgemäßen Verfahrens gespült. Nach dem Druckhaltetest wird extrakorporale Kreislauf wieder angekoppelt, ein venennahes Auslassventil geöffnet, bis die nun mit umgekehrtem Vorzeichen versehene Grenzschicht Blut/Substituat detektiert wird. Das ausströmende mikroblasenbelastete Substituat wird verworfen. Das Auslassventil wird nun geschlossen und die Blutreinigung fortgesetzt. Bei der räumlichen Entkoppelung wird ein weiteres Gefäß zur Zwischenspeicherung von Substituatlösung genutzt, aus welchem dann während des Druckhaltetests das blutseitige System gespeist wird. Ansonsten erfolgt die Verfahrensführung wie bei der zeitlichen Entkoppelung.

Unter Verwendung einer erfindungsgemäßen Dialysemaschine kann in beiden der gezeigten Ausführungsformen auch eine Anpassung der Pumpenrate auf eine optimale Geschwindigkeit während der Spülung erfolgen.

Eine solche Anpassung kann vor dem Hintergrund erfolgen, dass beobachtet wurde, dass insbesondere in den ersten Minuten der Behandlung ein hoher Eintrag von Mikroblasen in den Patienten stattfindet. Am Anfang kann der mittlere Mikroblasenfluss um eine Größenordnung höher sein als während der restlichen Behandlung. Außerdem gibt eine Reihe von Luftansammlungen im extrakorporalen Kreislauf, die als Quelle und Reservoir für spätere Mikroblaseninjektionen dienen können (Blutpumpenschlauch, Heparin- und andere Injektionsspritzen, zuführende Leitungen (z.B. Heparinschlauch und andere Ableitungen). So kann beispielsweise die Blut- oder Spülpumpenrate während der Spülung so betrieben werden, dass die Mikroblasen besser entfernt werden können. Die teilweise stationären Mikroblasen sollen z.B. mittels wiederholtem Stop-and-Go der Pumpe und hoher Pumpengeschwindigkeit abgelöst werden. Fluss- und Druckstöße greifen Luftansammlungen und Mikroblasenreservoire an und reduzieren diese. Diese Stöße können durch z.B. durch kurzzeitige maximale Pumpenraten, Sperren und Lösen einer venösen Klemme und dergleichen erreicht werden. Mittels des Ablaufs in der venösen Leitung werden die abgelösten Mirkoblasen aus dem extrakorporalen Kreislauf entfernt.

Ferner kann eine solche Anpassung vor dem Hintergrund erfolgen, dass beobachtet wurde, dass das rasche Anlaufen der Blutpumpe eine heftige Erhöhung der Mikroblasenzahl nach sich ziehen kann, sowie dass insbesondere ein Druckhaltetest, bei dem die Dialysierflüssigkeit nicht mehr erneuert wird und das Blut in den Dialysatorkapillaren nicht mehr mit frischer, entgaster Dialysierflüssigkeit umspült wird, eine heftige Erhöhung der Mikroblasenzahl nach sich ziehen kann. Deshalb kann die Blutpumpe nach der Spülung gegebenenfalls langsam hochfahren, z.B. in einer Rampe mit nicht mehr als 200 ml/min², was bedeutet, dass nach einem Blutpumpenstopp ein Sollrate von beispielsweise 400 ml/min erst nach 2 Minuten erreicht wird. Im Falle eines Druckhaltetests kann die Blutpumpenrate rechtzeitig vor dem Test abgesenkt werden, die Förderrate von Blutpumpe und Substitutionspumpe während der Spülung und des Druckhaltetests minimal bleiben, und beide Raten anschließend langsam wieder auf ihren Sollwert hochfahren.

Zusammenfassend kann festgehalten werden, dass es die Erfindung ermöglicht, zu einem beliebigen Zeitpunkt, beispielsweise während eines Druckhaltetests, einen Zielbereich des extrakorporalen Blutkreislaufs zu spülen und so die Bildung von Mirkoblasen zu vermeiden. Die Spülflüssigkeit kann über den separaten Ablauf verworfen werden.

## Patentansprüche

1. Verfahren zum Auswaschen von Gasblasen aus einem Zielbereich eines extrakorporalen Blutkreislaufs (5), vorzugsweise eines extrakorporalen Blutkreislaufs einer Dialysemaschine, wobei
der Zielbereich mit einer Spülflüssigkeit durchströmt wird, die durch einen Zulauf (14) in den Zielbereich eintritt und diesen durch einen Ablauf (15) wieder verlässt, und
der Zulauf (14) sich vom arteriellen Port (7) und der Ablauf (15) sich vom venösen Port (11) des extrakorporalen Blutkreislaufs (5) unterscheiden,
**dadurch gekennzeichnet, dass**
das Verfahren die folgenden Schritte umfasst, die in der dargestellten Reihenfolge (a)-(g) durchgeführt werden:
(a) Schließen einer arteriellen Klemme (21) und Öffnen des Zulaufs (14);
(b) Fördern eines ersten Volumens an Spülflüssigkeit in den Zielbereich, wobei das erste Volumen dem Volumen des Zielbereichs im Wesentlichen entspricht;
(c) Schließen einer venösen Klemme (22) und Öffnen des Ablaufs (15);
(d) Durchspülen des Zielbereichs mit der Spülflüssigkeit;
(e) Öffnen der arteriellen Klemme (21) und Schließen des Zulaufs (14);
(f) Ableiten eines zweiten Volumens an Spülflüssigkeit aus dem Zielbereich, wobei das zweite Volumen dem Volumen des Zielbereichs im Wesentlichen entspricht; und
(g) Schließen des Ablaufs (15) und Öffnen der venösen Klemme (22).

2. Verfahren nach Anspruch 1, **dadurch gekennzeichnet, dass** der Ablauf (15) in der venösen Leitung (10) angeordnet ist und/oder dass der Ablauf (15) stromabwärts aller bestehenden Schnittstellen des extrakorporalen Blutkreislaufs (5) angeordnet ist.

3. Verfahren nach einem der vorhergehenden Ansprüche, **dadurch gekennzeichnet, dass** der Zulauf (14) in der arteriellen Leitung (8) stromaufwärts oder stromabwärts der Blutpumpe (9) angeordnet ist.

4. Verfahren nach einem der vorhergehenden Ansprüche, **dadurch gekennzeichnet, dass** der extrakorporale Blutkreislauf (5) eine arterielle Klemme (21) und/oder eine venöse Klemme (22) umfasst, wobei die arterielle Klemme (21) vorzugsweise zwischen arteriellem Port (7) und Zulauf (14) und die venöse Klemme (22) vorzugsweise zwischen Ablauf (15) und venösem Port (11) angeordnet ist.

5. Verfahren nach einem der vorhergehenden Ansprüche, **dadurch gekennzeichnet, dass** der Zulauf (14) und/oder der Ablauf (15) anhand einer Klemme verschließbar ist oder anhand eines Dreiwegeventils mit dem extrakorporalen Blutkreislauf (5) verbunden ist.

6. Verfahren nach einem der vorhergehenden Ansprüche, **dadurch gekennzeichnet, dass** die Flussrate und/oder der Druck der Spülflüssigkeit im extrakorporalen Blutkreislauf (5) während des Spülvorganges wenigstens zeitweise inkonstant ist, und/oder dass die Flussrate der Spülflüssigkeit im extrakorporalen Blutkreislauf (5) während des Spülvorganges wenigstens zeitweise außerhalb eines Bereichs möglicher Flussraten liegt, der während der Behandlung für das Blut angewandt wird.

7. Verfahren nach einem der vorhergehenden Ansprüche, **dadurch gekennzeichnet, dass** das Verfahren während einer Behandlungsunterbrechung, insbesondere während eines Druckhaltetests durchgeführt wird.

8. Verfahren nach einem der vorhergehenden Ansprüche, **dadurch gekennzeichnet, dass** das Verfahren die folgenden Schritte umfasst:
(a') Anhalten der Blutpumpe (9) und Öffnen des Zulaufs (14);
(b') Fördern eines ersten Volumens an Spülflüssigkeit in den Zielbereich, wobei das erste Volumen dem Volumen des Zielbereichs im Wesentlichen entspricht;
(c') Schließen einer venösen Klemme (22) und Öffnen des Ablaufs (15);
(d') Durchspülen des Zielbereichs mit der Spülflüssigkeit;
(e') Schließen des Zulaufs (14) und Starten der Blutpumpe (9);
(f') Ableiten eines zweiten Volumens an Spülflüssigkeit aus dem Zielbereich, wobei das zweite Volumen dem Volumen des Zielbereichs im Wesentlichen entspricht; und
(g') Schließen des Ablaufs (15) und Öffnen der venösen Klemme (22).

9. Extrakorporale Blutbehandlungseinheit, vorzugsweise Dialysemaschine mit einem extrakorporalen Blutkreislauf (5) und einer Steuereinheit, wobei
der extrakorporale Blutkreislauf (5) einen Zulauf (14) und einen Ablauf (15) für Spülflüssigkeit aufweist, wobei sich der Zulauf (14) vom arteriellen Port (7) und der Ablauf (15) vom venösen Port (11) des extrakorporalen Blutkreislaufs (5) unterscheiden, und
die Steuereinheit ausgebildet ist, ein Verfahren gemäß einem der vorhergehenden Ansprüche durchzuführen.

10. Extrakorporale Blutbehandlungseinheit nach Anspruch 9, **dadurch gekennzeichnet, dass** die extrakorporale Blutbehandlungseinheit ein Leitungssystem für Spülflüssigkeit umfasst, das so an den extrakorporalen Blutkreislauf (5) angeschlossen ist, dass Spülflüssigkeit durch einen Zulauf (14) in den Zielbereich eintreten und diesen durch einen Ablauf (15) wieder verlassen kann, wobei das Leitungssystem vorzugsweise eine Spülpumpe umfasst, die stromaufwärts des Zulaufs (14) im Leitungssystem angeordnet ist.

11. Extrakorporale Blutbehandlungseinheit nach Anspruch 9 oder 10, **dadurch gekennzeichnet, dass** die Steuereinheit so ausgebildet ist, dass sie die Behandlung vor Durchführung des Verfahrens unterbricht und/oder dass sie das Verfahren während einer Unterbrechung der Behandlung auslöst und/oder dass sie die Behandlung nach Durchführung des Verfahrens automatisch fortsetzt.

12. Extrakorporale Blutbehandlungseinheit nach einem der Ansprüche 9 bis 11, **dadurch gekennzeichnet, dass** die Steuereinheit so ausgebildet ist, dass sie die Menge von durch das Verfahren zugeführter Spülflüssigkeit und/oder durch das Verfahren abgeleiteten Blutes bei der Ermittlung behandlungsspezifischer Parameter, insbesondere bei der Ermittlung des Ultrafiltrationsvolumens berücksichtigt.

13. Extrakorporale Blutbehandlungseinheit nach einem der Ansprüche 9 bis 12, **dadurch gekennzeichnet, dass** die Steuereinheit so ausgebildet ist, dass nach Abschluss des Verfahrens die Fördergeschwindigkeit für Blut langsam erhöht wird.

## Claims

1. A method of purging gas bubbles from a target zone of an extracorporeal blood circuit (5), preferably of an extracorporeal blood circuit of a dialysis machine, wherein
the target zone is flowed through by a flushing liquid which enters into the target zone through an inflow (14) and exits it again through an outflow (15), and
the inflow (14) differs from the arterial port (7) and the outflow (15) differs from the venous port (11) of the extracorporeal blood circuit (5),
**characterized in that**
the method comprises the following steps, which are conducted in the indicated order (a) - (g):
(a) Closing an arterial clamp (21) and opening the inflow (14);
(b) Conveying a first volume of flushing liquid into the target zone, with the first volume substantially corresponding to the volume of the target zone;
(c) Closing a venous clamp (22) and opening the outflow (15);
(d) Flushing through of the target zone with the flushing liquid;
(e) Opening the arterial clamp (21) and closing the inflow (14);
(f) Draining of a second volume of flushing liquid from the target zone, with the second volume substantially corresponding to the volume of the target zone; and
(g) Closing the outflow (15) and opening the venous clamp (22).

2. A method in accordance with claim 1, **characterized in that** the outflow (15) is arranged in the venous line (19) and/or **in that** the outflow (15) is arranged downstream of all existing interfaces of the extracorporeal blood circuit (5).

3. A method in accordance with one of the preceding claims, **characterized in that** the inflow (14) is arranged upstream or downstream of the blood pump (9) in the arterial line (8).

4. A method in accordance with one of the preceding claims, **characterized in that** the extracorporeal blood circuit (5) comprises an arterial clamp (21) and/or a venous clamp (22), with the arterial clamp (21) preferably being arranged between the arterial port (7) and the inflow (14), and with the venous clamp (22) preferably being arranged between the outflow (15) and the venous port (11).

5. A method in accordance with one of the preceding claims, **characterized in that** the inflow (14) and/or the outflow (15) can be closed using a clamp or is/are connected to the extracorporeal blood circuit (5) using a three-way valve.

6. A method in accordance with one of the preceding claims, **characterized in that** the flow rate and/or the pressure of the flushing liquid in the extracorporeal blood circuit (5) is/are inconstant at least at times during the flushing process, and/or **in that** the flow rate of the flushing liquid in the extracorporeal blood circuit (5) lies outside a range of possible flow rates at least at times during the flushing process, which range is used during the treatment for the blood.

7. A method in accordance with one of the preceding claims, **characterized in that** the method is carried out during a treatment interruption, in particular during a pressure holding test.

8. A method in accordance with one of the preceding claims, **characterized in that** the method comprises the following steps:
(a') Stopping the blood pump (9) and opening the inflow (14);
(b') Conveying a first volume of flushing liquid into the target zone, with the first volume substantially corresponding to the volume of the target zone;
(c') Closing a venous clamp (22) and opening the outflow (15);
(d') Flushing through of the target zone with the flushing liquid;
(e') Closing the inflow (14) and starting the blood pump (9);
(f') Draining of a second volume of flushing liquid from the target zone, with the second volume substantially corresponding to the volume of the target zone; and
(g') Closing the outflow (15) and opening the venous clamp (22).

9. An extracorporeal blood treatment unit, preferably a dialysis machine, having an extracorporeal blood circuit (5) and a control unit, wherein
the extracorporeal blood circuit (5) comprises an inflow (14) and an outflow (15) for flushing liquid, with the inflow (14) differing from the arterial port (7) and the outflow (15) differing from the venous port (11) of the extracorporeal blood circuit (5), and
with the control unit being configured to carry out a method in accordance with one of the preceding claims.

10. An extracorporeal blood treatment unit in accordance with claim 9, **characterized in that** the extracorporeal blood treatment unit comprises a line system for flushing liquid which is connected to the extracorporeal blood circuit (5) so that flushing liquid can enter into the target zone through an inflow (14) and can leave it again through an outflow (15), with the line system preferably comprising a flushing pump which is arranged upstream of the inflow (14) in the line system.

11. An extracorporeal blood treatment unit in accordance with claim 9 or 10, **characterized in that** the control unit is configured so that it interrupts the treatment before the carrying out of the method and/or so that it triggers the method during an interruption of the treatment and/or so that it automatically continues the treatment after the carrying out of the method.

12. An extracorporeal blood treatment unit in accordance with one of the claims 9 to 11, **characterized in that** the control unit is configured so that it takes account of the quantity of flushing liquid supplied by the method and/or of the blood drained off by the method in the determination of treatment-specific parameters, in particular in the determination of the ultrafiltration volume.

13. An extracorporeal blood treatment unit in accordance with one of the claims 9 to 12, **characterized in that** the control unit is configured so that the conveying speed for blood is slowly increased after the end of the process.

## Revendications

1. Procédé servant à éliminer par lavage des bulles de gaz d'une zone cible d'un circuit de sang extracorporel (5), de préférence d'un circuit de sang extracorporel d'une machine de dialyse, dans lequel
la zone cible est traversée par un liquide de rinçage, qui entre par une entrée (14) dans la zone cible et quitte à nouveau celle-ci par une sortie (15), et l'entrée (14) se distingue de l'orifice artériel (7) et la sortie (15) se distingue de l'orifice veineux (11) du circuit de sang extracorporel (5),
**caractérisé en ce que**
le procédé comprend les étapes suivantes qui sont mises en oeuvre dans l'ordre (a) - (g) illustré :
(a) la fermeture d'une bride de serrage artérielle (21) et l'ouverture de l'entrée (14) ;
(b) le refoulement d'un premier volume de liquide de rinçage dans la zone cible, dans lequel le premier volume correspond sensiblement au volume de la zone cible ;
(c) la fermeture d'une bride de serrage veineuse (22) et l'ouverture de la sortie (15) ;
(d) le rinçage complet de la zone cible avec le liquide de rinçage ;
(e) l'ouverture de la bride de serrage artérielle (21) et la fermeture de l'entrée (14) ;
(f) l'évacuation d'un deuxième volume de liquide de rinçage hors de la zone cible, dans lequel le deuxième volume correspond sensiblement au volume de la zone cible ; et
(g) la fermeture de la sortie (15) et l'ouverture de la bride de serrage veineuse (22).

2. Procédé selon la revendication 1, **caractérisé en ce que** la sortie (15) est disposée dans le conduit veineux (10), et/ou que la sortie (15) est disposée en aval de toutes les interfaces existantes du circuit de sang extracorporel (5).

3. Procédé selon l'une quelconque des revendications précédentes, **caractérisé en ce que** l'entrée (14) est disposée dans le conduit artériel (8) en amont ou en aval de la pompe à sang (9).

4. Procédé selon l'une quelconque des revendications précédentes, **caractérisé en ce que** le circuit de sang extracorporel (5) comprend une bride de serrage artérielle (21) et/ou une bride de serrage veineuse (22), dans lequel la bride de serrage artérielle (21) est disposée de préférence entre un orifice artériel (7) et l'entrée (14) et la bride de serrage veineuse (22) est disposée de préférence entre la sortie (15) et l'orifice veineux (11).

5. Procédé selon l'une quelconque des revendications précédentes, **caractérisé en ce que** l'entrée (14) et/ou la sortie (15) peuvent être fermées à l'aide d'une bride de serrage ou sont reliées à l'aide d'une soupape à trois voies au circuit de sang extracorporel (5).

6. Procédé selon l'une quelconque des revendications précédentes, **caractérisé en ce que** le débit et/ou la pression du liquide de rinçage dans le circuit de sang extracorporel (5) sont inconstants au moins par intermittence pendant l'opération de rinçage, et/ou que le débit du liquide de rinçage dans le circuit de sang extracorporel (5) se situe pendant l'opération de rinçage au moins par intermittence en dehors d'une plage de débits éventuels, qui est appliquée pendant le traitement pour le sang.

7. Procédé selon l'une quelconque des revendications précédentes, **caractérisé en ce que** le procédé est mis en oeuvre pendant une interruption de traitement, en particulier pendant un test de maintien de la pression.

8. Procédé selon l'une quelconque des revendications précédentes, **caractérisé en ce que** le procédé comprend les étapes suivantes :
(a') l'arrêt de la pompe à sang (9) et l'ouverture de l'entrée (14) ;
(b') le refoulement d'un premier volume de liquide de rinçage dans la zone cible, dans lequel le premier volume correspond sensiblement au volume de la zone cible ;
(c') la fermeture d'une bride de serrage veineuse (22) et l'ouverture de la sortie (15) ;
(d') le rinçage complet de la zone cible avec le liquide de rinçage ;
(e') la fermeture de l'entrée (14) et le démarrage de la pompe à sang (9) ;
(f') l'évacuation d'un deuxième volume de liquide de rinçage hors de la zone cible, dans lequel le deuxième volume correspond sensiblement au volume de la zone cible ; et
(g') la fermeture de la sortie (15) et l'ouverture de la bride de serrage veineuse (22).

9. Unité de traitement de sang extracorporelle, de préférence machine de dialyse avec un circuit de sang extracorporel (5) et une unité de commande, dans laquelle
le circuit de sang extracorporel (5) présente une entrée (14) et une sortie (15) pour du liquide de rinçage, dans laquelle l'entrée (14) se distingue de l'orifice artériel (7) et la sortie (15) se distingue de l'orifice veineux (11) du circuit de sang extracorporel (5), et
l'unité de commande est réalisée pour mettre en oeuvre un procédé selon l'une quelconque des revendications précédentes.

10. Unité de traitement du sang extracorporelle selon la revendication 9, **caractérisée en ce que** l'unité de traitement du sang extracorporelle comprend un système d'acheminement pour du liquide de rinçage, qui est raccordé de telle sorte au circuit de sang extracorporel (5) que du liquide de rinçage peut entrer par une entrée (14) dans la zone cible et peut quitter à nouveau celle-ci par une sortie (15), dans laquelle le système d'acheminement comprend de préférence une pompe de rinçage, qui est disposée en amont de l'entrée (14) dans le système d'acheminement.

11. Unité de traitement du sang extracorporelle selon la revendication 9 ou 10, **caractérisée en ce que** l'unité de commande est réalisée de telle sorte qu'elle interrompt le traitement avant la mise en oeuvre du procédé et/ou qu'elle déclenche le procédé pendant une interruption du traitement et/ou qu'elle poursuit automatiquement le traitement après la mise en oeuvre du procédé.

12. Unité de traitement du sang extracorporelle selon l'une quelconque des revendications 9 à 11, **caractérisée en ce que** l'unité de commande est réalisée de telle sorte qu'elle tient compte de la quantité de liquide de rinçage amené par le procédé et/ou de sang évacué par le procédé lors de la détermination de paramètres spécifiques au traitement, en particulier lors de la détermination du volume d'ultrafiltration.

13. Unité de traitement du sang extracorporelle selon l'une quelconque des revendications 9 à 12, **caractérisée en ce que** l'unité de commande est réalisée de telle sorte à l'issue du procédé, la vitesse de refoulement du sang est augmentée lentement.
